(19) Europäisches Patentamt
European Patent Office
Office européen des brevets

(11) EP 0 858 314 B1

(12) EUROPÄISCHE PATENTSCHRIFT

(45) Veröffentlichungstag und Bekanntmachung des Hinweises auf die Patenterteilung:
**17.07.2002 Patentblatt 2002/29**

(21) Anmeldenummer: **96937269.7**

(22) Anmeldetag: **28.10.1996**

(51) Int Cl.[7]: **A61K 7/06**

(86) Internationale Anmeldenummer:
**PCT/EP96/04683**

(87) Internationale Veröffentlichungsnummer:
**WO 97/17051 (15.05.1997 Gazette 1997/21)**

(54) **HAARBEHANDLUNGSMITTEL**

HAIR-TREATMENT AGENTS

PRODUITS DE TRAITEMENT CAPILLAIRE

(84) Benannte Vertragsstaaten:
**AT BE CH DE DK ES FI FR GB GR IT LI NL PT SE**

(30) Priorität: **03.11.1995 DE 19540853**

(43) Veröffentlichungstag der Anmeldung:
**19.08.1998 Patentblatt 1998/34**

(73) Patentinhaber: **Henkel Kommanditgesellschaft auf Aktien
40589 Düsseldorf-Holthausen (DE)**

(72) Erfinder:
- **HOLLENBERG, Detlef
D-40699 Erkrath (DE)**
- **EHLERT, Manuela
D-51379 Leverkusen (DE)**
- **GODDINGER, Dieter
D-42799 Leichlingen (DE)**

(56) Entgegenhaltungen:
**EP-A- 0 446 636**
**WO-A-91/14418**
**WO-A-96/03969**
**DE-A- 4 324 962**
**DE-C- 899 248**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Haarbehandlungsmittel mit einer speziellen Wirkstoffkombination sowie Verfahren zur Behandlung von Haaren unter Verwendung dieser Mittel.

Das menschliche Haupthaar wird heute in vielfältiger Weise mit haarkosmetischen Zubereitungen behandelt. Dazu gehört beispielsweise die Reinigung der Haare mit Shampoos und Duschpräparaten und das Bleichen, Färben und Verformen von Haaren mit Wellmitteln, Tönungsmitteln und Stylingpräparaten. Als Folge fast aller Haarbehandlungen kann es zu unerwünschten Beeinträchtigungen der Haarstruktur kommen. Diese Beeinträchtigungen zeigen sich u.a. in einer schlechten Naß- und Trockenkämmbarkeit, einer verstärkten elektrostatischen Aufladung, verstärkter Sprödigkeit, verringerter Höchstreißkraft und Reißdehnung der Haare sowie einem verschlechterten äußeren Erscheinungsbild der Frisur. Weiterhin können beispielsweise Färbemittel in einigen Fällen zu noch mit dem bloßen Auge erkennbar ungleichmäßigen Ausfärbungen führen.

Es hat daher nicht an Versuchen gefehlt, diese Mißstände zu bekämpfen. Dabei wurden u.a. spezielle Nachbehandlungsmittel entwickelt, die insbesondere zur Verbesserung der Struktur und der Eigenschaften geschädigter Haare, kurz gesagt zur Wiederherstellung der physikalischen Eigenschaften gesunder Haare dienen. Weiterhin wurden Wirkstoffkombinationen gefunden, deren Einarbeitung in bekannte Haarbehandlungsmittel den genannten Haarschädigungen in möglichst großem Umfang vorbeugen sollen, oder auf andere Weise das Erscheinungsbild der behandelten Haare verbessern. Als Beispiel für eine solche Verbesserung seien hier nur egalisierende Bestandteile von Haarfärbemitteln genannt.

Gegenstand der DE-A1-40 09 617 sind beispielsweise Mittel zur Pflege strapazierter Haare mit einem Gehalt an kationischen, oberflächenaktiven Verbindungen, die eine Wirkstoff-Kombination aus Polyvinylpyrrolidon und wasserlöslichen Oligo- oder Polypeptiden enthalten. Dadurch wird insbesondere die Festigkeit des Haare erhöht. Wenngleich dadurch hervorragende Ergebnisse auf dem Haar erzeilt werden, läßt doch die wegen der Staubbildung nicht ganz problemlose Einarbeitbarkeit des PVP noch Verbesserungswünsche offen.

Gegenstand der WO 92/17153 sind Zubereitungen zur Behandlung von Haaren, die ein kationisch derivatisiertes Proteinhydrolysat und ein Kohlenhydrat und/oder ein kationisches, anionisches oder Ampho-Polymer enthalten. Durch Einsatz dieser Wirkstoffkombinationen wird sowohl die Reißfestigkeit des Haares erhöht als auch ein besserer Halt der Frisur und ein verbessertes Volumen erhalten.

Gegenstand der EP 0 446 636-A2 sind Mittel für die äußere Anwendung auf menschlicher oder tierischer Haut, die ein Polymer oder Copolymer des N-Vinylacetamids oder ein vernetztes Produkt daraus als essentielle Komponente enthält.

Das Dokument WO 96/03969 offenbart gemäß dem Anspruch 8 ein Haarbehandlungsmittel in Form eines Gels, welches Gelbildner enthält, die ausgesucht sind aus der Gruppe der synthetischen Polymere, den Cellulose-Verdickungsmitteln, den Verdickungsmitteln auf Stärkebasis und den natürlich vorkommenden Gummen.

Es besteht aber weiterhin ein Bedarf an haarkosmetischen Zubereitungen, die sich durch eine Verringerung der unerwünschten Beeinträchtigungen der Haare auszeichnen und gleichzeitig weitere positive Effekte bezüglich des Erscheinungsbildes des Haares (z.B. Egalisierung der Ausfärbung colorierter Haare) zeigen.

Es wurde nunmehr gefunden, daß die Verwendung bestimmter nichtionogener Polymerer in Kombination mit mindestens einem weiteren spezifischen Wirkstoff in Haarbehandlungsmitteln zu überraschend guten Ergebnissen führt. Insbesondere wird überraschenderweise eine ausgeprägte festigende Wirkung erzielt, die dem Haar Halt und Fülle gibt.

Gegenstand der Erfindung ist ein Haarbehandlungsmittel enthaltend übliche kosmetische Bestandteile, das dadurch gekennzeichnet ist, daß es ein nichtionogenes Polymer enthaltend Einheiten der Formel (I),

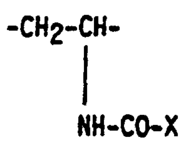

in der X steht für Wasserstoff, eine lineare oder verzweigte A1-kylgruppe mit 1 bis 22 Kohlenstoffatomen oder eine ein- oder mehrfach ungesättigte Alkenylgruppe mit 2 bis 22 Kohlenstoffatomen,
in Kombination mit mindestens einem weiteren Wirkstoff, ausgewählt aus

- der Gruppe (A), die aus Aminosäuren, Oligopeptiden und Polypeptiden sowie deren Derivaten besteht, und/oder

- der Gruppe (B) der Kohlenhydrate, wobei das Kohlenhydrat nicht ausgewählt ist aus Verdickungsmitteln auf Cellulose- oder Stärkebasis sowie in der Natur vorkommenden Gummen,
  und/oder
- der Gruppe (C), die aus Panthenol sowie nichtionogenen und kationischen Derivaten des Panthenols besteht,
  enthält, mit den Maßgaben für den Fall, daß das Mittel keine Wirkstoffe aus den Gruppen (B) und (C) enthält, daß der Wirkstoff der Gruppe (A) nicht ausgewählt ist aus den hydrolysierten Proteinen des Kollagens oder Keratins und daß die Aminosäure als alleiniger Wirkstoff aus der Gruppe (A) ausgewählt ist aus Glutaminsäure, Asparaginsäure und Valin.

Die erste zwingend enthaltene Komponente der erfindungsgemäßen Haarbehandlungsmittel sind nichtionogene Polymere mit Einheiten der Formel (I). Bevorzugt sind mindestens 50 %, insbesondere mindestens 80 % der Einheiten der nichtionogenen Polymere solche der Formel (I). Nichtionogene Polymere, die - von herstellungsbedingten Verunreinigungen sowie Endgruppen abgesehen - ausschließlich aus Einheiten der Formel (I) aufgebaut sind, haben sich als erfindungsgemäß besonders geeignet erwiesen.
In der Formel (I) steht X für

- Wasserstoff,
- lineare oder verzweigte Alkylgruppen mit 1-22 Kohlenstoffatomen, wie beispielweise Methyl-, Ethyl-, iso-Propyl-, n-Propyl-, n-Butyl-, iso-Butyl, n-Pentyl-, n-Hexyl-, n-Decyl-, Lauryl-, Myristyl-, Cetyl-, Stearyl- und iso-Stearylgruppen,
- ein- oder mehrfach ungesättigte Alkenylgruppen mit 2-22 Kohlenstoffatomen, wie beispielsweise Vinyl-, Allyl-, Oleyl- und Linoleylgruppen.

Verbindungen, bei denen X für Wasserstoff oder eine $C_1$-$C_4$-Gruppe steht, sind besonders geeignet. Unter diesen sind wiederum das Poly(N-vinyl-acetamid) sowie insbesondere das Poly(N-vinyl-formamid) bevorzugt. Die vorteilhafte Verwendung von Poly(N-vinylformamid) in Haarstyling-Produkten ist aus der Druckschrift Parfümerie und Kosmetik, 76 (10), 616-619 (1995) bekannt. Diese Druckschrift gibt aber ebenso wenig Hinweise auf die erfindungsgemäßen Kombinationen wie die nach dem Prioritätstag der vorliegenden Anmeldung veröffentlichte WO 96/03969.

Geeignete nichtionische Co-Monomere, die mit Einheiten der Formel (I) in den erfindungsgemäß verwendeten Polymeren kombiniert werden können, sind beispielsweise Vinylpyrrolidon, Vinylester wie Vinylacetat, Acrylamid, Methacrylamid, Acrylsäureester wie Methylacrylat und Ethylacrylat, Methacrylsäureester wie Methylmethacrylat und Ethylmethacrylat sowie Vinylalkohol.

Die Molmasse der nichtionogenen Polymeren liegt üblicherweise in einem Bereich von 10.000 bis 1.500.000 Dalton; Polymere mit Molmassen zwischen 200.000 und 500.000 Dalton sind besonders geeignet.

Desgleichen werden solche nichtionogenen Polymere bevorzugt, die wasserlöslich, d.h. bei Raumtemperatur zu mindestens 0,1, insbesondere zu mindestens 2 Gew.-% in Wasser löslich sind.

Das nichtionogene Polymer ist in den erfindungsgemäßen Haarbehandlungsmitteln bevorzugt in einer Menge von 0,01 bis 10 Gew.-%, insbesondere 0,1 bis 2 Gew-%, bezogen auf das gesamte Mittel, enthalten.

Weiterhin enthalten die erfindungsgemäßen Haarbehandlungsmittel mindestens einen weiteren Wirkstoff, ausgewählt aus den oben definierten Gruppen (A), (B) und (C).

Die Gruppe (A) wird gebildet aus Aminosäuren, Oligopeptiden und Polypeptiden sowie deren Derivaten.

Erfindungsgemäß einsetzbare Aminosäuren sind beispielsweise Glutaminsäure, Asparaginsäure, Arginin, Lysin, Cystein, Cystin, Glutamin, Asparagin und Valin. Glutaminsäure, Asparaginsäure, Serin und Valin haben sich als besonders wirksam erwiesen. Ebenfalls als erfindungsgemäß geeignet haben sich Aminosäuregemische erwiesen, wie sie durch vollständige basische, saure oder enzymatische Hydrolyse von tierischen oder pflanzlichen Proteinen erhalten werden können.

Erfindungsgemäß einsetzbare Oligopeptide und Polypeptide sind beispielsweise tierische oder pflanzliche Proteine oder deren auf saurem, basischem oder enzymatischen Wege gewonnenen (Teil-)Hydrolysate. Geeignete Proteine sind beispielsweise Keratin, Kollagen, Elastin, Sojaprotein, Milchprotein, Casein, Fibroine, Sericin, Weizenprotein, Seidenprotein und Mandelprotein. Keratin sowie die pflanzlichen Proteine können erfindungsgemäß bevorzugt sein. Durch die Hydrolyse entstehen Stoffmischungen mit mittleren Molmassen im Bereich von ca. 400 bis ca. 50 000 Dalton. Übliche mittlere Molmassen liegen in einem Bereich von etwa 500 bis etwa 8000 Dalton. Hydrolysate von Keratin und von pflanzlichen Proteinen sind erfindungsgemäß bevorzugt.

Unter Derivaten der Aminosäuren, Oligopeptide und Polypeptide werden erfindungsgemäß deren kationische Derivate sowie deren Kondensationsprodukte mit Fettsäuren verstanden.

Kationische Derivate erhält man durch Umsetzung mit Verbindungen, die üblicherweise quartäre Ammoniumgruppen tragen oder durch Umsetzung mit entsprechenden Aminen und anschließende Quaternierung.

Eine Reihe solcher quartärer Proteinhydrolysate sind als Handelsprodukte erhältlich, beispielsweise:

- kationisches Kollagenhydrolysat, beispielsweise das unter der Bezeichnung Lamequat$^{R}$L auf dem Markt befindliche Produkt (INCI-Bezeichnung: Lauryldimonium Hydroxypropyl Hydrolyzed Collagen; Chemische Fabrik Grünau) mit der Struktur

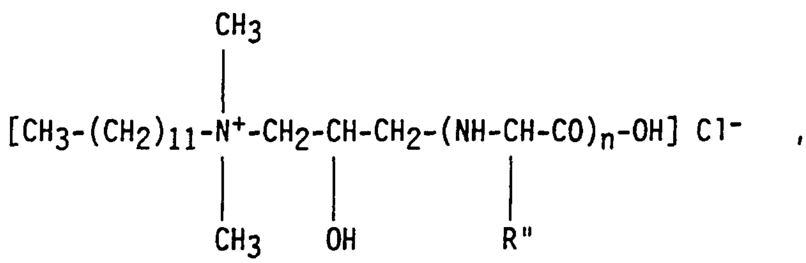

in der R" für die Seitenketten der Aminosäuren des Kollagens steht.

- kationisches Keratinhydrolysat, beispielsweise das unter der Bezeichnung Croquat$^{R}$ auf dem Markt befindliche Produkt (CTFA-Bezeichnung: Cocodimonium Hydroxypropyl Hydrolyzed Keratin; Croda)

- kationisches Weizenhydrolysat, erhältlich unter der Bezeichnung Hydrotriticum$^{R}$QL (CTFA-Bezeichnung: Lauryldimonium Hydroxypropyl Hydrolyzed Wheat Protein; Croda)

- das unter der Bezeichnung Crotein$^{R}$Q erhältliche Produkt, gemäß INCI ein "Hydroxypropyltrimonium Hydrolyzed Collagen" (Croda) sowie

- das als Lexein$^{R}$Q X 3000 (Inolex) erhältliche quaternierte Eiweißhydrolysat.

Zur Herstellung der Kondensationsprodukte von Proteinhydrolysaten mit Fettsäuren werden als Säurekomponente bevorzugt Ölsäure, Myristinsäure, Undecylensäure, Kokosfettsäure und Abietinsäure verwendet. Diese Kondensationsprodukte können auch in Form von Salzen, insbesondere Natrium-, Kalium- und Triethanolaminsalzen vorliegen.

Solche Kondensationsprodukte auf Basis Kollagenhydrolysat tragen auch die CTFA-Bezeichnungen Oleoyl Hydrolyzed Collagen, Myristoyl Hydrolyzed Collagen, Oleoyl Hydrolyzed Animal Collagen, Potassium Coco Hydrolyzed Animal Protein, TEA Abietoyl Hydrolyzed Collagen, Potassium Undecylenoyl Hydrolyzed Collagen und TEA Coco Hydrolyzed Collagen. Handelsprodukte sind beispielsweise Lamepon$^{R}$LPO, Lamepon$^{R}$4 SK, Lamepon$^{R}$UD, Lamepon$^{R}$460, Lamepon$^{R}$PA TR, Lamepon$^{R}$ST 40 und Lamepon$^{R}$S (Grünau) sowie Lexein$^{R}$A 240, Lexein$^{R}$S 620 und LexeinRA 520 (Inolex).

Kondensationsprodukte von Elastinhydrolysaten mit Fettsäuren wie beispielsweise Laurinsäure (CTFA-Bezeichnung: Lauroyl Hydrolyzed Elastin) können ebenfalls eingesetzt werden. Crolastin$^{R}$AS (Croda) ist ein entsprechendes Marktprodukt.

Unter der Bezeichnung Promois EGCP erhältlich ist ein Potassium Cocoyl Hydrolyzed Wheat Protein (Seiwa).

Weitere erfindungsgemäß einsetzbare Handelsprodukte sind Lexein$^{R}$A 200 (Inolex), Lamepon$^{R}$PO-TR, Lamepon$^{R}$PA-K, Lamepon$^{R}$S-MV und Lamepon$^{R}$S-TR (Grünau) und Crotein$^{R}$CCT (Croda).

Die Gruppe (B) besteht aus Kohlenhydraten.

Als Kohlenhydrate können erfindungsgemäß sowohl Monosaccharide als auch Oligosaccharide, wie beispielweise Rohrzucker, Milchzucker und Raffinose, eingesetzt werden. Die Verwendung von Monosacchariden ist bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose und Fructose. Arabinose, Glucose, Galactose und Fructose sind bevorzugt eingesetzte Kohlenhydrate; Glucose ist ganz besonders bevorzugt.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zukkeralkohole sind Sorbit, Mannit und Dulcit. Bevorzugte Glykoside sind die Methylglucoside.

Quaternierte Kohlenhydrate können ebenfalls erfindungsgemäß als Komponente (B) eingesetzt werden. Das

Handelsprodukt Glucquat[R]100 (entsprechend der CTFA-Nomenklatur ein Lauryl Methyl Gluceth-10 Hydroxypropyl Dimonium Chloride) ist ein bevorzugtes quaterniertes Kohlenhydrat.

Da die eingesetzten Kohlenhydrate üblicherweise aus natürlichen Rohstoffen wie Stärke gewonnen werden, weisen die Kohlenhydrate in der Regel die diesen Rohstoffen entsprechenden Konfigurationen auf (z.B. D-Glucose, D-Fructose und D-Galactose).

Die Gruppe (C) besteht aus Panthenol sowie nichtionogenen und kationischen Derivaten des Panthenols.

Geeignete nichtionoge Derivate des Panthenols sind beispielsweise Pantothenylethylether und Pantothensäure. Erfindungsgemäß geeignete kationische Derivate des Panthenols sind beispielsweise Verbindungen der allgemeinen Formel

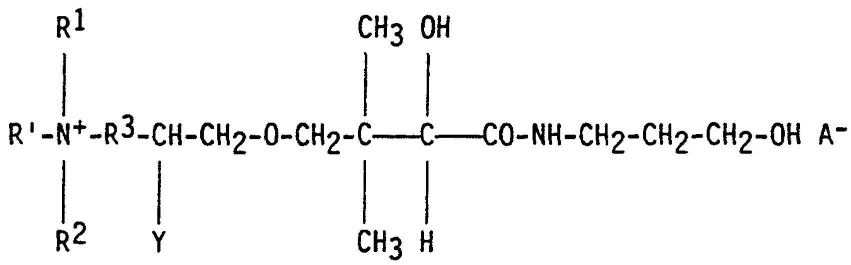

,

in der R', $R^1$ und $R^2$ unabhängig voneinander für eine Alkylgruppe mit 1-24 Kohlenstoffatomen oder eine Alkenylgruppe mit 8-24 Kohlenstoffatomen, $R^3$ für eine Alkylengruppe mit 1-18 Kohlenstoffatomen, Y für eine OH-Gruppe oder für Wasserstoff und A für ein einwertiges, organisches oder anorganisches Anion steht.

Solche Verbindungen sind beispielsweise aus der PCT-Offenlegungsschrift WO 92/13829 bekannt als kosmetische Wirkstoffe, insbesondere für Haarbehandlungsmittel.

Bevorzugt sind dabei solche Verbindungen, bei denen R' für eine Alkyl- oder Alkenylgruppe mit 8-24 Kohlenstoffatomen steht. Solche Alkyl- bzw. Alkenylgruppen sind beispielsweise Lauryl-, Myristyl-, Cetyl-, Stearyl- und Oleyl-Gruppen. $R^1$ und $R^2$ sind bevorzugt Alkylgruppen mit 1-4 Kohlenstoffatomen, insbesondere Methylgruppen. $R^3$ ist ebenfalls bevorzugt eine kürzere Alkylengruppe mit 1-4 Kohlenstoffatomen, insbesondere eine Methylengruppe. Y steht, herstellungsbedingt, bevorzugt für eine Hydroxygruppe, A für ein Halogenidion, insbesondere für Chlorid. Ein entsprechendes Produkt wird von der Firma Tri-K unter der Bezeichnung PANTHEQUAT[R] vertrieben.

Die weiteren Wirkstoffe aus den Gruppen (A) bis (C) sind in den erfindungsgemäßen Haarbehandlungsmitteln bevorzugt in einer Menge von 0,01 bis 10 Gew.-%, insbesondere 0,1 bis 5 Gew-%, bezogen auf das gesamte Mittel, enthalten.

Die weiteren Bestandteile der erfindungsgemäßen Haarbehandlungsmittel sind von der Art des Haarbehandlungsmittels abhängig. Dabei kann die erfindungsgemäße Wirkstoffkombination ein wesentliches Wirkprinzip des speziellen Haarbehandlungsmittels sein; die Wirkstoffkombination kann aber auch in Mittel eingearbeitet werden, die primär einem anderen Zweck, beispielsweise der Reinigung oder der Tönung der Haare, dienen.

Prinzipiell umfassen die erfindungsgemäßen Formulierungen aber alle bekannten Arten von Haarbehandlungsmitteln wie z.B. Haarshampoos, Haarspülungen, Haarkonditioniermittel, Haarkuren, Haarfestiger, Haarsprays, Fönwellen, Dauerwellmittel und Haarfärbemittel. Haarkuren und Haarkonditioniermittel, insbesondere solche Mittel, die morgens auf das Haar aufgetragen werden, um diesen für den weiteren Verlauf des Tages eine bestimmte Festigung zu geben, sowie Haarshampoos stellen bevorzugte Formen der erfindungsgemäßen Mittel dar.

Abhängig von der Art des Haarbehandlungsmittels können die erfindungsgemäßen Zubereitungen alle in solchen Mitteln bekannten Wirk-, Zusatz- und Hilfsstoffe enthalten. In vielen Fällen enthalten die Zubereitungen mindestens ein Tensid, wobei prinzipiell sowohl anionische als auch zwitterionische, ampholytische, nichtionische und kationische Tenside geeignet sind. In vielen Fällen hat es sich dabei als vorteilhaft erwiesen, die Tenside aus den nichtionischen Tensiden auszuwählen.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare und verzweigte Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,

- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Als bevorzugte nichtionische Tenside haben sich die Alkylenoxid-Anlagerungsprodukte an gesättigte lineare Fettalkohole und Fettsäuren mit jeweils 4 bis 20 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

In einer weiteren Ausführungsform kann es bevorzugt sein, als nichtionische Tenside Alkylpolyglykoside der allgemeinen Formel $RO-(Z)_x$ auszuwählen. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare und verzweigte Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-OSO_3H$, in der R eine bevorzugt lineare Alkylgruppe mit 10 bis 18 C-Atomen und x = 0 oder 1 bis 12 ist,

- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO(-)- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammonium-chlorid und Tricetylmethylammoniumchlorid.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil$^R$-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid$^R$S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die Produkte Dehyquart$^R$ AU-46, Dehyquart$^R$F-30 und Dehyquart$^R$F-75 sowie die unter dem Warenzeichen Stepantex$^R$ vertriebenen Dialkylammoniummethosulfate und Methyl-hydroxyalkyl-dialkoyloxyalkyl-ammoniummethosulfate.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside sind in den erfindungsgemäßen Mitteln üblicherweise in Mengen von 0,1 bis 65 Gew.-%, bevorzugt in Mengen von 2 bis 50 Gew.-% und ganz besonders bevorzugt in Mengen von 8 bis 20 Gew.-%, bezogen auf das gesamte Mittel, enthalten. Niotenside sind bevorzugt in Mengen von 0,1 bis 10 Gew.-%, insbesondere in Mengen von 0,5 bis 5 Gew.-%, enthalten.

In einer weiteren bevorzugten Ausführungsform enthalten die erfindungsgemäßen Zubereitungen zusätzlich

noch einen haarkonditionierenden Wirkstoff. Bevorzugte haarkonditionierende Wirkstoffe können die bereits oben aufgeführten kationischen Tenside sein.

Eine weitere bevorzugte Gruppe von haarkonditionierenden Wirkstoffen sind Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.

Eine weitere Gruppe haarkonditionierender Wirkstoffe sind kationische Polymere.

Die erfindungsgemäß verwendbaren kationischen Polymeren enthalten innerhalb des Polymergerüstes kationische Gruppen. Diese Gruppen können Teil der Polymerkette sein; sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliniumgruppen.

Eine Reihe von für die Haarpflege geeigneten kationischen Polymeren sind dem Fachmann bekannt und als Handelsprodukte erhältlich.

Beispiele für solche Polymeren sind:

- quaternierte Cellulose-Derivate, wie sie unter den Bezeichnungen Celquat$^R$ und Polymer JR$^R$ im Handel erhältlich sind. Die Verbindungen Celquat H 100, Celquat L 200 und Polymer JR$^R$400 sind bevorzugte quaternierte Cellulose-Derivate.

- quaternierte Guar-Derivate, wie sie unter den Bezeichnungen Cosmedia Guar$^R$ und Jaguar$^R$ im Handel erhältlich sind. Bevorzugte Guar-Derivate sind beispielsweise Cosmedia Guar$^R$ C-261 und Jaguar$^R$ C 13-S.

- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere sowie das Vinylpyrrolidon-Methacrylamidopropyltrimethylammoniumchlorid-Copolymere. Solche Verbindungen sind unter den Bezeichnungen Gafquat$^R$734, Gafquat$^R$755 bzw. Gafquat$^R$ HS100 im Handel erhältlich.

- Copolymerisate des Vinylpyrrolidons mit Vinylimidazoliummethochlorid, wie sie unter der Bezeichnung Luviquat$^R$ angeboten werden.

- Polymere Dimethyldiallylammoniumsalze und deren Copolymere mit Estern und Amiden von Acrylsäure und Methacrylsäure. Die unter den Bezeichnungen Merquat$^R$100 (Poly(dimethyldiallylammoniumchlorid)) und Merquat$^R$550 (Dimethyldiallylammoniumchlorid-Acrylamid-Copolymer) im Handel erhältlichen Produkte sind Beispiele für solche kationischen Polymere.

- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil$^R$-Quat 3270 und 7232 (Hersteller: Th. Goldschmidt; diquaternäre Polydimethylsiloxane, Quaternium-80).

- Chitosan und dessen Derivate

Schließlich können auch Phospholipide, beispielsweise Sojalecithin, Ei-Lecithin und Kephaline als haarkonditionierende Wirkstoffe eingesetzt werden.

Bevorzugt sind die haarkonditionierenden Wirkstoffe in Mengen von 0,1 - 5 Gew.-%, bezogen auf die gesamte Zubereitung, enthalten.

Die erfindungsgemäßen Haarbehandlungsmittel können neben den oben genannten nichtionogenen Polymeren gewünschtenfalls auch noch weitere nichtionogene Polymere enthalten.

Geeignete nichtionogene Polymere sind beispielsweise:

- Vinylpyrrolidon/Vinylester-Copolymere, wie sie beispielsweise unter dem Warenzeichen Luviskol$^R$ (BASF) vertrieben werden. LuviskolR VA 64 und Luviskol$^R$ VA 73, jeweils Vinylpyrrolidon/Vinylacetat-Copolymere, sind ebenfalls bevorzugte nichtionische Polymere.

- Celluloseether, wie Hydroxypropylcellulose, Hydroxyethylcellulose und Methylhydroxypropylcellulose, wie sie beispielsweise unter den Warenzeichen Culminal$^R$ und Benecel$^R$ (AQUALON) vertrieben werden.

- Schellack

- Polyvinylpyrrolidone, wie sie beispielsweise unter der Bezeichnung Luviskol$^R$ (BASF) vertrieben werden.

Üblicherweise sind solche weiteren nichtionogenen Polymere aber gegenüber den Polymeren mit den Einheiten gemäß Formel (I) in untergeordneten Mengen eingesetzt. Eine Ausnahme bilden lediglich solche Polymere, die gleichzeitig die Funktion eines Verdickungsmittels zur Einstellung der Viskosität ausüben.

Eine weitere fakultative Komponente der erfindungsgemäßen Haarbehandlungsmittel stellen anionische Polymere dar, von denen dem Fachmann eine Vielzahl von Handelsprodukten bekannt sind. Als Beispiele werden genannt:

- Copolymere der Acrylsäure und/oder Methacrylsäure oder deren Ester mit $C_{10-30}$-Alkylacrylaten, wie sie beispielsweise unter der Bezeichnung Pemulen$^R$ vertrieben werden.

- Polymere und Copolymere der Crotonsäure mit Estern und Amiden der Acryl- und der Methacrylsäure, wie Vinylacetat-Crotonsäureund Vinylacetat-Vinylpropionat-Crotonsäure-Copolymere. Verbindungen dieser Art sind unter den Markenbezeichnungen Resyn$^R$ (NATIONAL STARCH), Luviset$^R$ (BASF) und Gafset$^R$ (GAF) im Handel; die Produkte Luviset$^R$CA-66 und Luviset$^R$CAP können bevorzugt sein.

- Vinylpyrrolidon/Vinylacrylat-Copolymere, erhältlich beispielsweise unter dem Warenzeichen Luviflex$^R$ (BASF). Ein bevorzugtes Polymer ist das unter der Bezeichnung Luviflex$^R$ VBM-35 (BASF) erhältliche Vinylpyrrolidon/Acrylat-Terpolymere.

- Acrylsäure/Ethylacrylat/N-tert.Butylacrylamid-Terpolymere, die beispielsweise unter der Bezeichnung Ultrahold$^R$ strong (BASF) vertrieben werden, sowie Methacrylsäure/Ethylacrylat/t-Butylacrylat-Terpolymer, die unter der Bezeichnung Luvimer$^R$100P (BASF) vertrieben werden.

Weitere Bestandteile der erfindungsgemäßen Mittel können beispielsweise sein:

- Strukturanten wie Maleinsäure,
- Verdickungsmittel wie Agar-Agar, Guar-Gum, Alginate und Xanthan-Gum,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine, Zink Omadine und Climbazol,
- weitere Substanzen zur Einstellung des pH-Wertes,
- Wirkstoffe wie Allantoin, Pyrrolidoncarbonsäuren und deren Salze, Pflanzenextrakte und Vitamine,
- Lichtschutzmittel,
- Konsistenzgeber wie Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, EDETA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und $\alpha$-Mercaptoethansulfonsäure,
- Oxidationsmittel wie Wasserstoffperoxid, Kaliumbromat und Natriumbromat.

Die erfindungsgemäß verwendbaren Mittel können als Lösung, Lotion, Emulsion, Mikroemulsion, Creme oder

Gel formuliert sein. Die Formulierung als Lösung, Emulsion oder Mikroemulsion mit einem Wassergehalt von 50 bis 90 Gew.-%, bezogen auf das gesamte Mittel, kann bevorzugt sein.

In einer weiteren bevorzugten Ausführungsform können die Mittel in Form von Schaumaerosolen konfektioniert werden. Dabei ist sowohl die Formulierung mit einem verflüssigten Gas als Treibmittel als auch in Form sogenannter Pumpsprays möglich, bei denen der zum Versprühen benötigte Druck durch mechanisches Pumpen aufgebaut wird. Stickstoff, Luft, Kohlendioxid, Propan, Butan, Isobutan, Pentan und Dimethylether sind bevorzugte Treibmittel. Wenngleich Fluorchlor- und Chlorkohlenwasserstoffe hinsichtlich der erzielten Aerosoleigenschaften exzellente Treibmittel sind, so ist doch ihre Verwendung als Treibmittel in den erfindungsgemäßen Mitteln aufgrund der bekannten Ozon-Problematik weniger bevorzugt.

Gegenstand der Erfindung ist auch die Verwendung der erfindungsgemäßen Mittel zur Behandlung von Keratinfasern, insbesondere von Haaren.

Gegenstand der Erfindung ist ebenfalls ein Verfahren zur Behandlung von Haaren, bei dem eine erfindungsgemäße Zubereitung auf das Haar aufgebracht wird und dort verbleibt.

Gegenstand der Erfindung ist schließlich auch ein Verfahren zur Behandlung von Haaren, bei dem eine erfindungsgemäße Zubereitung auf das Haar aufgebracht und nach einer Einwirkzeit (0,5 bis 40 Minuten) wieder ausgespült wird.

Die folgenden Beispiele sollen die Erfindung näher erläutern.

## Beispiele

### 1. Anwendungsbeispiele

Alle Mengenangabe in den folgenden Anwendungsbeispielen sind in Gew.-%.

**1. Kurpackung (auf dem Haar verbleibend)**

| | |
|---|---|
| Poly(N-vinyl-formamid)[1] | 1,0 |
| Nutrilan Keratin W[2] | 2,5 |
| Cetiol^R OE[3] | 0,6 |
| Eumulgin^R 05[4] | 0,4 |
| D-Panthenol | 0,6 |
| Ethanol | 10,0 |
| Glucose | 0,1 |
| Carbopol^R ETD 2001[5] | 0,6 |
| Triethanolamin | q.s. |
| Wasser | ad 100 |

[1] Mittl. Molmasse: 350.000 (NATIONAL STARCH)
[2] Hydrolysat aus Merinoschurwolle (INCI-Bezeichnung: Hydrolized Keratin; 20 % Aktivsubstanz) (HENKEL)
[3] Dioctylether (INCI-Bezeichnung: Dicaprylyl Ether) (HENKEL)
[4] Oleylalkohol + 5 Ethylenoxid (INCI-Bezeichnung: Oleth-5) (HENKEL)
[5] vernetzte Polyacrylsäure (INCI-Bezeichnung: Carbomer) (GOODRICH)

**2. Shampoo**

| | |
|---|---|
| Poly(N-vinyl-formamid) | 0,7 |
| Sorbit | 0,5 |
| Lamequat^R L[6] | 2,9 |
| Texapon^R N 70[7] | 21,0 |
| Plantaren^R-1200[8] | 8,0 |
| Genamin^R DSAC[9] | 1,2 |

[6] kationisiertes Kollagenhydrolysat (35 % Aktivsubstanz in Wasser; INCI-Bezeichnung: Lauryldimonium Hydroxypropyl Hydrolyzed Collagen) (HENKEL)
[7] Natriumlaurylethersulfat (ca. 72 % Aktivsubstanz) (HENKEL)
[8] C12-C16-Alkylglucosid mit Oligomerisationsgrad 1,4 (ca. 50 % Aktivsubstanz; INCI-Bezeichnung: Lauryl Polyglycosid) (HENKEL)
[9] Dimethyldistearylammoniumchlorid (HOECHST)

(fortgesetzt)

| | |
|---|---|
| Cutina[R]EGMS[10] | 0,6 |
| Natriumchlorid | 0,2 |
| Wasser | ad 100 |

10 Ethylenglykolmonostearat (ca. 25-35% Monoester, 60-70% Di- ester; INCI-Bezeichnung: Glycol Stearate) (HENKEL)

**3. Kurpackung (abspülbar)**

| | |
|---|---|
| Poly(N-vinyl-formamid) | 0,5 |
| Glutaminsäure | 0,2 |
| Stenol[R]1618[11] | 3,0 |
| Eumulgin[R]B 1[12] | 0,5 |
| Eumulgin[R]B 2[13] | 0,5 |
| Cutina[R]CP[14] | 1,0 |
| Eutanol[R]G[15] | 1,5 |
| Dow Corning[R]929-Emulsion[16] | 2,9 |
| Celquat[R]L200[17] | 2,0 |
| Carbopol[R]ETD 2050[18] | 0,7 |
| Lamepon[R] PA-K[19] | 3,0 |
| Natronlauge (25%ig) | q.s. |
| Wasser | ad 100 |

11 C16/C18-Fettalkohol (HENKEL)

12 Cetylstearylalkohol mit ca. 12 Mol E0 (INCI-Bezeichnung: Ceteareth-12) (HENKEL)

13 Cetylstearylalkohol mit ca. 20 Mol E0 (INCI-Bezeichnung: Ceteareth-20) (HENKEL)

14 Ester aus gesättigten, langkettigen Fettalkoholen und Fettsäuren, vornehmlich Palmitinsäurecetylester (INCI-Bezeichnung: Cetyl Palmitate) (HENKEL)

15 Kondensationsprodukt aus gesättigten flüssigen Fettalkoholen, vorwie- gend Decylalkohol, hergestellt nach der Guerbet-Reaktion (INCI-Bezeichnung: Octyldodecanol) (HENKEL)

16 amino-funktionelles Polydimethylsiloxan (35 % Aktivsubstanz) (DOW CORNING)

17 Hydroxyethylcellulose-Diallyldimethylammoniumchlorid-Copolymere (95 % Aktivsubstanz; INCI-Bezeichnung: Polyquaternium-4) (DELFT-NATIONAL)

18 modifizierte Polyacrylsäure (INCI-Bezeichnung: Carbomer) (GOODRICH)

19 Eiweiß-Abietinsäure-Kondensat, Kalium-Salz (INCI-Bezeichung: Potassium Abietoyl Hydrolyzed Collagen; ca. 31 % Aktivsubstanz) (HENKEL)

**4. Spülung**

| | |
|---|---|
| Paraffinöl(perliquidum) | 3,96 |
| Cetyl-Stearylalkohol | 4,8 |
| Glycerin-monostearat-/palmitat | 0,54 |
| Fettalkohol ($C_{12-18}$)-Ethoxylat(10 EO) | 0,48 |
| Distearyl-dimethyl-ammonium-chlorid | 0,2 |
| Cetyltrimethylammoniumchlorid(25 %ig in $H_2O$) | 3,0 |
| Glucose-Monohydrat | 1,0 |
| Methyl-hydroxypropylcellulose (3 %ig in $H_2O$) | 20 |
| Poly(N-vinylformamid) | 2,0 |
| PHB-Methylester | 0,2 |
| Elastinhydrolysat (20 %ig in $H_2O$) | 10 |
| Wasser | ad 100 |

**5. Kurpackung (abspülbar)**

| | |
|---|---|
| Paraffinöl (perliquidum) | 3,96 |
| Cetyl-Stearylalkohol | 4,8 |
| Glycerin-monostearat-/palmitat | 0,54 |

(fortgesetzt)

| | |
|---|---|
| Fettalkohol ($C_{12-18}$)-Ethoxylat (10 EO) | 0,48 |
| Cetyltrimethylammonium-chlorid (25 %ig in $H_2O$) | 3,0 |
| Glucose-Monohydrat | 1,0 |
| Methyl-hydroxypropylcellulose (3 %ig in $H_2O$) | 20 |
| Poly(N-vinyl-formamid) | 2,0 |
| PHB-Methylester | 0,2 |
| PHB-Propylester | 0,2 |
| Phenoxyethanol | 0,5 |
| Duftstoff | 0,1 |
| Plantasol W 20[20] | 10 |
| Wasser | ad 100 |

[20] Weizenproteinhydrolysat (ca. 18 % Aktivsubstanz; INCI-Bezeichnung: Hydrolyzed Wheat Protein) (DEUTSCHE GELATINE FABRIKEN STOESS AG)

**6. Kurpackung (abspülbar)**

| | |
|---|---|
| Paraffinöl (perliquidum) | 3,96 |
| Cetyl-Stearylalkohol | 4,8 |
| Glycerin-monostearat-/palmitat | 0,54 |
| Fettalkohol ($C_{12-18}$)-Ethoxylat (10 EO) | 0,48 |
| Cetyltrimethylammonium-chlorid (25 %ig in $H_2O$) | 3,0 |
| Mannose | 1,0 |
| Methyl-hydroxypropylcellulose (3 %ig in $H_2O$) | 20 |
| Poly(N-vinyl-formamid) | 2,0 |
| PHB-Methylester | 0,2 |
| PHB-Propylester | 0,2 |
| Phenoxyethanol | 0,5 |
| Duftstoff | 0,1 |
| Collagenhydrolysat (35,5 % in $H_2O$) | 6,0 |
| Wasser | ad 100 |

**7. Kurpackung (abspülbar)**

| | |
|---|---|
| Paraffinöl (perliquidum) | 3,96 |
| Cetyl-Stearylalkohol | 4,8 |
| Glycerin-monostearat-/palmitat | 0,54 |
| Fettalkohol ($C_{12-18}$)-Ethoxylat (10 EO) | 0,48 |
| Cetyltrimethylammonium-chlorid (25 %ig in $H_2O$) | 3,0 |
| Glucose-Monohydrat | 1,0 |
| Methyl-hydroxypropylcellulose (3 %ig in $H_2O$) | 20 |
| Poly(N-vinyl-formamid) | 2,0 |
| PHB-Methylester | 0,2 |
| PHB-Propylester | 0,2 |
| Phenoxyethanol | 0,5 |
| Duftstoff | 0,1 |
| Elastinhydrolysat (20 %ig in $H_2O$) | 10 |
| Wasser | ad 100 |

## 2. Anwendungstechnische Untersuchungen

### 2.1. Meßverfahren

#### 2.1.1. "Curl Retention"-Test

Die Messungen wurde an Haartressen (# 6921 der Firma Fischbach und Miller; je 2 g Masse und 16 cm Länge) bei 21 °C und 75 % rel. Luftfeuchtigkeit durchgeführt.
0,2 g des zu untersuchenden Mittels wurden auf die nasse Haartresse aufgebracht und einmassiert. Sodann wurde die feuchte Tresse auf Spiralwickel (3 cm Durchmesser) aufgewickelt, 3 Stunden bei 60 °C getrocknet und dann über Nacht bei 21 °C gelagert. Danach wurde die Locke abgewickelt und 6 Stunden lang bei 21 °C und 75 % relativer Luftfeuchtigkeit ausgehängt.
Der "Curl Retention"-Wert ergibt sich dann aus der Beziehung

$$c_r = (l - l_6) / (l - l_o) * 100 \%.$$

Dabei bedeuten:

- $l$: Länge der Haartresse (16 cm)
- $l_o$: Länge der Haarlocke von der Nähnaht bis zur Unterkante direkt nach dem Abwickeln
- $l_6$: Länge der Haarlocke von der Nähnaht bis zur Unterkante nach 6 Stunden Aushängen.

#### 2.1.2. Volumenmessung nach der "Schattenmethode"

Eine unbehandelte Haarsträhne (#6923 der Firma Fischbach und Miller; je 2g Masse und 15 cm Länge) wurden mit einer Aniontensidlösung (50 Gew.-% Texapon$^R$N 25 (Natriumlaurylether(2 EO)sulfat; 25 % Aktivsubstanz; HENKEL) in Wasser) shampooniert und mit Wasser ausgespült. Dann wurden 0,3 g der Probelösung auf die nasse Haarsträhne aufgetragen und einmassiert. Sodann wurde die Haarsträhne an einem Stativ befestigt und jeweils 30mal, zunächst mit einem Kamm und dann mit einer Rundbürste, gekämmt, wobei jeweils mit einem Fön getrocknet wurde. Dann wurde die Strähne noch 15 Minuten zum weiteren Trocknen an dem Stativ belassen.
Anschließend wurden die Konturen der Strähne auf einem Papier nachgezeichnet. Sodann wurde der Durchmesser dieser Kontur auf Höhe der Strähnenmitte auf dem Papier mit einem Lineal vermessen. Die Größe dieses Durchmessers ist ein Maß für das "Volumen" der Haarsträhne.

### 2.2. Ergebnisse

Die untersuchten Mischungen sowie die gemessenen "Curl Retention"-Werte (jeweils Mittelwerte aus 4 Bestimmungen) und die Werte aus der Volumenmessung nach der "Schattenmethode" sind in der folgenden Tabelle aufgeführt.

| Zubereitung | E1 | V1 | V2 | V3 | V4 | V5 |
|---|---|---|---|---|---|---|
| Komponenten [g]: | | | | | | |
| - Poly-(N-vinyl-formamid) | 1,0 | - | 1,0 | - | - | - |
| - Luviskol$^R$K 30[21] | - | - | - | 1,0 | 1,0 | - |
| - Panthenol | 0,5 | - | - | - | 0,5 | 0,5 |
| - Glucose | 1,0 | - | - | - | 1,0 | 1,0 |
| - Plantasol W 20 | 0,5 | - | - | - | 0,5 | 0,5 |
| - Wasser | < ---------- ad 100 ---------- > | | | | | |
| $c_r$ [%] | 70 | 58 | 61 | 64 | 63 | 51 |
| Durchmesser [cm] ("Schattenmethode") | 6,2 | 4,2 | 5,0 | 5,3 | 4,7 | 3,1 |

[21] Polyvinylpyrrolidon (BASF)

**Patentansprüche**

1. Haarbehandlungsmittel enthaltend übliche kosmetische Bestandteile, **dadurch gekennzeichnet, daß** es zusätzlich ein nichtionogenes Polymer mit Einheiten der Formel (I),

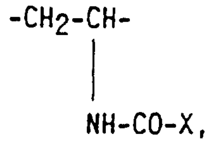

in der X steht für Wasserstoff, eine lineare oder verzweigte Alkylgruppe mit 1 bis 22 Kohlenstoffatomen oder eine ein- oder mehrfach ungesättigte Alkenylgruppe mit 2 bis 22 Kohlenstoffatomen,
in Kombination mit mindestens einem weiteren Wirkstoff, ausgewählt aus

- der Gruppe (A), die aus Aminosäuren, Oligopeptiden und Polypeptiden sowie deren Derivaten besteht, und/oder
- der Gruppe (B) der Kohlenhydrate, wobei das Kohlenhydrat nicht ausgewählt ist aus Verdickungsmitteln auf Cellulose- oder Stärkebasis sowie in der Natur vorkommenden Gummen,
und/oder
- der Gruppe (C), die aus Panthenol sowie nichtionogenen und kationischen Derivaten des Panthenols besteht,

enthält, mit den Maßgaben für den Fall, daß das Mittel keine Wirkstoffe aus den Gruppen (B) und (C) enthält, daß der Wirkstoff der Gruppe (A) nicht ausgewählt ist aus den hydrolysierten Proteinen des Kollagens oder Keratins und daß die Aminosäure als alleiniger Wirkstoff aus der Gruppe (A) ausgewählt ist aus Glutaminsäure, Asparaginsäure und Valin.

2. Haarbehandlungsmittel nach Anspruch 1, **dadurch gekennzeichnet, daß** das nichtionogene Polymer ausschließlich aus Einheiten der Formel (I) aufgebaut ist.

3. Haarbehandlungsmittel nach Anspruch 1 oder 2, **dadurch gekennzeichnet, daß** in der Formel (I) X für Wasserstoff oder einen Alkylrest mit 1 bis 4 Kohlenstoffatomen steht.

4. Haarbehandlungsmittel nach einem der Ansprüche 1 bis 3, **dadurch gekennzeichnet**, das der Wirkstoff aus der Gruppe (A) ausgewählt ist aus Hydrolysaten pflanzlicher Proteine oder deren Derivaten.

5. Haarbehandlungsmittel nach Anspruch 4, **dadurch gekennzeichnet, daß** das Proteinhydrolysat-Derivat ein Fettsäure-Proteinhydrolysat-Kondensat oder ein kationisch derivatisiertes Proteinhydrolysat ist.

6. Haarbehandlungsmittel nach einem der Ansprüche 1 bis 3, **dadurch gekennzeichnet, daß** die Aminosäure ausgewählt ist aus Glutaminsäure, Asparaginsäure, Serin und Valin.

7. Haarbehandlungsmittel nach einem der Ansprüche 1 bis 6, **dadurch gekennzeichnet, daß** das Kohlenhydrat ausgewählt ist aus Mono- und Disacchariden.

8. Haarbehandlungsmittel nach Anspruch 1 bis 7, **dadurch gekennzeichnet, daß** das Kohlenhydrat 5 oder 6 Kohlenstoffatomen enthält.

9. Haarbehandlungsmittel nach Anspruch 8, **dadurch gekennzeichnet, daß** das Kohlenhydrat Glucose ist.

10. Haarbehandlungsmittel nach einem der Ansprüche 1 bis 9, **dadurch gekennzeichnet, daß** der Wirkstoff der Gruppe (C) Panthenol ist.

11. Haarbehandlungsmittel nach einem der Ansprüche 1 bis 10, **dadurch gekennzeichnet, daß** es weiterhin ein nichtionogenes, anionisches, zwitterionisches, ampholytisches oder kationisches Tensid enthält.

**12.** Haarbehandlungsmittel nach einem der Ansprüche 1 bis 11, **dadurch gekennzeichnet, daß** das nichtionogene Polymere mit Einheiten der Formel (I) in einer Menge von 0,01 bis 10 Gew.-%, insbesondere 0,1 bis 2 Gew-%, bezogen auf das gesamte Mittel, enthalten ist.

**13.** Haarbehandlungsmittel nach einem der Ansprüche 1 bis 12, **dadurch gekennzeichnet, daß** der weitere Wirkstoff in einer Menge von 0,01 bis 10 Gew.-%, insbesondere 0,1 bis 5 Gew-%, bezogen auf das gesamte Mittel, enthalten ist.

**14.** Verwendung eines Haarbehandlungsmittel nach einem der Ansprüche 1 bis 13 zur Behandlung von Haaren.

**15.** Verfahren zur Behandlung von Haaren, **dadurch gekennzeichnet, daß** eine Zubereitung nach einem der Ansprüche 1 bis 13 auf das Haar aufgebracht wird und nach einer Einwirkzeit wieder ausgespült wird.

**16.** Verfahren zur Behandlung von Haaren, **dadurch gekennzeichnet, daß** eine Zubereitung nach einem der Ansprüche 1 bis 13 auf das Haar aufgebracht wird und dort verbleibt.

## Claims

**1.** A hair treatment composition containing typical cosmetic ingredients, **characterized in that** it additionally contains a nonionic polymer with units corresponding to formula (I):

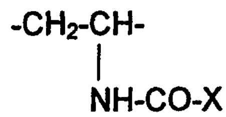

in which X stands for hydrogen, a linear or branched alkyl group containing 1 to 22 carbon atoms or a mono- or polyunsaturated alkenyl group containing 2 to 22 carbon atoms,
in combination with at least one other active principle selected from

- group (A) which consists of amino acids, oligopeptides and polypeptides and derivatives thereof
  and/or
- group (B) which consists of carbohydrates, the carbohydrate not being selected from cellulose- or starch-based thickeners or naturally occurring gums
  and/or
- group (C) which consists of panthenol and nonionic and cationic derivatives of panthenol,

with the provisos that, where the composition does not contain any active principles from groups (B) and (C), the group (A) active principle is not selected from hydrolyzed proteins of collagen or keratin and that the amino acid as sole group (A) active principle is selected from glutamic acid, aspartic acid and valine.

**2.** A hair treatment composition as claimed in claim 1, **characterized in that** the nonionic polymer is made up exclusively of units corresponding to formula (I).

**3.** A hair treatment composition as claimed in claim 1 or 2, **characterized in that**, in formula (I), X stands for hydrogen or $C_{1-4}$ alkyl group.

**4.** A hair treatment composition as claimed in any of claims 1 to 3, **characterized in that** the group (A) active principle is selected from hydrolyzates of vegetable proteins or derivatives thereof.

**5.** A hair treatment composition as claimed in claim 4, **characterized in that** the protein hydrolyzate derivative is a fatty acid protein hydrolyzate condensate or a cationically derivatized protein hydrolyzate.

**6.** A hair treatment composition as claimed in any of claims 1 to 3, **characterized in that** the amino acid is selected

from glutamic acid, aspartic acid, serine and valine.

**7.** A hair treatment composition as claimed in any of claims 1 to 6, **characterized in that** the carbohydrate is selected from mono- and disaccharides.

**8.** A hair treatment composition as claimed in claims 1 to 7, **characterized in that** the carbohydrate contains 5 or 6 carbon atoms.

**9.** A hair treatment composition as claimed in claim 8, **characterized in that** the carbohydrate is glucose.

**10.** A hair treatment composition as claimed in any of claims 1 to 9, **characterized in that** the group (C) active principle is panthenol.

**11.** A hair treatment composition as claimed in any of claims 1 to 10, **characterized in that** it additionally contains a nonionic, anionic, zwitterionic, ampholytic or cationic surfactant.

**12.** A hair treatment composition as claimed in any of claims 1 to 11, **characterized in that** the nonionic polymer containing units corresponding to formula (I) is present in a quantity of 0.01 to 10% by weight and more particularly in a quantity of 0.1 to 2% by weight, based on the composition as a whole.

**13.** A hair treatment composition as claimed in any of claims 1 to 12, **characterized in that** the other active principle is present in a quantity of 0.01 to 10% by weight and more particularly in a quantity of 0.1 to 5% by weight, based on the composition as a whole.

**14.** The use of the hair treatment composition claimed in any of claims 1 to 13 for treating hair.

**15.** A hair treatment process, **characterized in that** the preparation claimed in any of claims 1 to 13 is applied to the hair and is rinsed out again after a contact time.

**16.** A hair treatment process, **characterized in that** the preparation claimed in any of claims 1 to 13 is applied to the hair and is left thereon.

**Revendications**

**1.** Froduit de traitement capillaire contenant des composants cosmétiques usuels,
**caractérisé en ce qu'**
il contient un polymère non ionique contenant des motifs de formule (I)

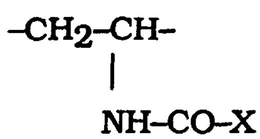

dans laquelle X représente un atome d'hydrogène, un groupe alkyle linéaire ou ramifié ayant de 1 à 22 atomes de carbone, ou un groupe alcényle une ou plusieurs fois insaturé, ayant de 2 à 22 atomes de carbone,
en association avec au moins une autre substance active choisie dans

- le groupe (A) qui consiste en aminoacides, oligopeptides et polypeptides ainsi que leurs dérivés, et/ou
- le groupe (B) des glucides, le glucide n'étant pas choisi parmi des épaississants à base de cellulose ou d'amidon ni des gommes existant dans la nature, et/ou
- le groupe (C) qui consiste en panthénol ainsi qu'en dérivés non ioniques et dérivés cationiques du panthénol,

étant entendu que pour le cas où le produit ne contient pas de substances actives des groupes (B) et (C), la substance active du groupe (A) n'est pas choisie parmi les protéines hydrolysées du collagène où de la kératine et que l'aminoacide, en tant que seule substance active du groupe (A), est choisi parmi l'acide glutamique, l'acide aspartique et la valine.

2. Produit de traitement capillaire selon la revendication 1,
**caractérisé en ce que**
le polymère non ionique est exclusivement constitué de motifs de formule (I).

3. Produit de traitement capillaire selon la revendication 1 ou 2,
**caractérisé en ce que**
dans la formule (I) X représente un atome d'hydrogène ou un radical alkyle ayant de 1 à 4 atomes de carbone.

4. Produit de traitement capillaire selon l'une quelconque des revendications 1 à 3,
**caractérisé en ce que**
la substance active du groupe (A) est choisie parmi des hydrolysats de protéine végétale ou leurs dérivés.

5. Produit de traitement capillaire selon la revendication 4,
**caractérisé en ce que**
le dérivé d'hydrolysat de protéine est un produit de condensation d'hydrolysat de protéine et d'acides gras ou un hydrolysat de protéine transformé en dérivé cationique.

6. Produit de traitement capillaire selon l'une quelconque des revendications 1 à 3,
**caractérisé en ce que**
l'aminoacide est choisi parmi l'acide glutamique, l'acide aspartique, la sérine et la valine.

7. Produit de traitement capillaire selon l'une quelconque des revendications 1 à 6.
**caractérisé en ce que**
le glucide est choisi parmi des mono- et disaccharides.

8. Produit de traitement capillaire selon l'une quelconque des revendications 1 à 7,
**caractérisé en ce que**
le glucide contient 5 ou 6 atomes de carbone.

9. Produit de traitement capillaire selon la revendication 8,
**caractérisé en ce que**
le glucide est le glucose.

10. Produit de traitement capillaire selon l'une quelconque des revendications 1 à 9,
**caractérisé en ce que**
la substance active du groupe (C) est le panthénol.

11. Produit de traitement capillaire selon l'une quelconque des revendications 1 à 10,
**caractérisé en ce qu'**
il contient en outre un tensioactif non ionique, anionique, zwitterionique, ampholyte ou cationique.

12. Produit de traitement capillaire selon l'une quelconque des revendications 1 à 11,
**caractérisé en ce que**
le polymère non ionique comportant des motifs de formule (I) est contenu en une quantité de 0,01 à 10% en poids, en particulier de 0,1 à 2 % en poids, par rapport au produit total.

13. Produit de traitement capillaire selon l'une quelconque des revendications 1 à 12,
**caractérisé en ce que**
l'autre substance active est contenue en une quantité de 0,01 à 10 % en poids, en particulier de 0,1 à 5 % en poids, par rapport au produit total.

14. Utilisation d'un produit de traitement capillaire selon l'une quelconque des revendications 1 à 13, pour le traitement des cheveux.

15. Procédé pour le traitement des cheveux,
**caractérisé en ce qu'**
une préparation selon l'une quelconque des revendications 1 à 13 est appliquée sur le cheveu et, après un temps d'action, est de nouveau éliminée par rinçage.

**16.** Procédé pour le traitement des cheveux, **caractérisé en ce qu'** une préparation selon l'une quelconque des revendications 1 à 13 est appliquée sur le cheveu et y reste.